# FASCICULE DE BREVET EUROPEEN

(11) **EP 0 549 779 B1**
(45) Date de publication et mention de la délivrance du brevet: **20.03.1996**
(21) Numéro de dépôt: 92916212.1
(22) Date de dépôt: 10.07.1992
(51) Int. Cl.: A61F 2/08, A61B 17/11

(54) **DISPOSITIF DE RENFORCEMENT D'UN LIGAMENT AU COURS D'UNE PLASTIE LIGAMENTAIRE**
VORRICHTUNG ZUM VERSTÄRKEN EINES LIGAMENTTRANSPLANTATS IN DER WIEDERHERSTELLUNGSCHIRURGIE
DEVICE FOR REINFORCING A LIGAMENT TRANSPLANT DURING RECONSTRUCTIVE SURGERY

(30) Priorité: 11.07.1991 FR 9108925
(43) Date de publication de la demande: 07.07.1993
(73) Titulaire: Legrand, Jean-Jacques, F-73000 Chambery (FR)
(72) Inventeur: Legrand, Jean-Jacques, F-73000 Chambery (FR)
(74) Mandataire: Dénoyez, Hubert
(86) Numéro de dépôt international: FR9200677
(87) Numéro de publication internationale: WO9300866

(56) Documents cités:
- EP-A- 0 328 401
- WO-A-82/04390
- US-A- 4 469 101
- US-A- 4 741 330
- US-A- 4 997 433

## Description

La présente invention a trait à la chirurgie orthopédique et plus particulièrement à la reconstruction ligamentaire des différentes articulations.

Les lésions ligamentaires sont à l'heure actuelle de plus en plus fréquentes, du fait des accidents de la route, de sport et du travail.

Dans la plupart des cas ces lésions consistent en une rupture du ligament, et il convient alors de procéder à une reconstruction ligamentaire.

Dans le cas d'une lésion fraîche, une suture et une réinsertion du ligament avec renforcement permettent en général de restaurer le ligament avec ou sans immobilisation, selon l'articulation concernée.

Dans le cas d'une lésion non décelée rapidement, les fragments de ligament se rétractent et se nécrosent, ce qui rend la suture impossible et nécessite de recourir à une plastie ligamentaire.

Cette plastie ligamentaire peut être effectuée au moyen soit d'un ligament artificiel ou prothétique, soit d' un ligament autologue provenant du prélèvement d'un transplant de voisinage, tendon ou ligament, soit encore d'un tendon de banque prélevé sur le corps d'un donneur.

Le ligament artificiel présente les avantages de la simplicité de mise en oeuvre et d'une solidité initiale ne nécessitant pas d'immobilisation, source de nombreux problèmes de circulation et de blocage des articulations, permettant ainsi de procéder à une rééducation immédiate.

Par contre il présente des inconvénients liés aux matériaux utilisés, à savoir des risques de ruptures et une certaine intolérance. Ces inconvénients expliquent que le ligament artificiel soit relativement peu utilisé.

On préfère ainsi utiliser soit des ligaments autologues, soit des ligaments ou tendons de banque, mais pour éviter une trop longue immobilisation, c'est-à-dire le temps que les ligaments soient bien ancrés et revascularisés, ce qui peut nécessiter 3 à 8 mais selon la localisation articulaire, on procède au renforcement dudit ligament au moyen soit d'un ligament artificiel avec les aléas de tolérance, soit de matériaux résorbables, en polymères d'acide lactique et/ou d'acide glycolique, bien tolérés car largement utilisés comme fils de suture.

Ces matériaux résorbables se présentent sous la forme de cordelette ou de ruban. Dans la pratique, on prélève une bande de ligament que l'on enroule dans le sens transversal autour d'une cordelette ou d'un ruban en matériau résorbable, et l'on fixe l'ensemble par les deux extrémités aux points d'insertion du ligament à remplacer. Dans le cas d'une lésion fraîche, un ruban en matériau résorbable est fixé parallèlement au ligament, ou tendon, suturé, de manière à le consolider.

Toutefois ces rubans et cordelettes sont mal adaptés au rôle qu'on leur demande de jouer : en effet du fait des caractéristiques mécaniques différentes du ligament et du matériau résorbable, lesdits rubans ou cordelettes pontent les forces exercées sur l'articulation, et de ce fait le ligament n'est pas sollicité, ou très peu, et lors de la résorption desdits rubans et cordelettes, le ligament qui n'a jamais, ou peu, travaillé, peut soit être distendu soit risquer la rupture.

Afin de remédier à cet inconvénient on a proposé, dans EP-A-0328401, un dispositif de renforcement d'un ligament réalisé en un matériau résorbable, et présentant les caractéristiques figurant dans le préambule de la revendication 1.

Le dispositif objet de la présente invention comprend un manchon tricoté dans un fil en matériau résorbable, destiné à envelopper le transplant ou le tendon de banque. Le point de tricot utilisé est de ceux connus permettant audit manchon d'être extensible dans le sens diamétral et peu dans le sens longitudinal quand le diamètre est fixé : c'est en effet la compression possible du transplant qui assure l'élasticité de l'ensemble transplant matériau-résorbable, et non le fil lui-même.

De plus le maillage est calculé en dimensions de façon à autoriser les échanges biologiques entre le transplant ou le tendon de banque et le milieu environnant, os, synoviale, tissu cellulaire sous-cutané, etc...

Enfin le léger frottement du manchon autour du transplant ou du tendon de banque permet une stimulation périphérique et un massage de celui-ci lui permettant de recréer des fibres orientées dans différents sens pour assurer son travail.

Le manchon en fil résorbable peut être réalisé dans des diamètres et des longueurs différents afin de correspondre aux ligaments de toutes les articulations, et il est conditionné sur un applicateur constitué d'un tube de matière souple susceptible d'être aplati selon deux génératrices diamétralement opposées, permettant un rangement aisé.

Selon un premier mode de réalisation du dispositif selon l'invention, le manchon comporte à l'une de ses extrémités deux boucles permettant la solidarisation, au moyen d'agrafes, dudit manchon à l'un des points d'insertion, son autre extrémité étant, après adaptation de la longueur, agrafée avec le transplant ou le tendon de banque, à l'autre point d'insertion.

Lors de son utilisation, il suffit, en exerçant une pression sur les plis de l'applicateur, de redonner à celui-ci, et au manchon, la forme d'un cylindre, et d'introduire dans ledit applicateur le transplant ou le tendon de banque. Après enlèvement de l'applicateur, en exerçant une traction sur les deux extrémités du manchon, le diamètre de celui-ci s'adapte à celui du transplant ou du tendon de banque, il suffit alors de couper ledit manchon à la bonne longueur, du côté ne comportant pas les deux boucles de fixation.

Dans le cas d'une lésion fraîche, il suffit d'engager le manchon en fil résorbable sur un des segments du ligament ou du tendon à suturer, d'abouter les deux segments de ligament ou de tendon et de les suturer, puis de faire coulisser ledit manchon sur toute la longueur du ligament ou tendon suturé et d'en solidariser les extrémités.

Selon un second mode de réalisation du dispositif selon l'invention, applicable au cas où le tendon de banque doit être accroché à l'os, tel celui d'une intervention sur le ligament croisé du genou, le manchon est associé à un dispositif permettant de réaliser l'ancrage d'une de ses extrémités dans une perforation réalisée dans l'os où il doit venir s'accrocher.

Le dispositif mis en oeuvre dans ce second mode de réalisation se compose d'un tube à tête ogivale au sommet de laquelle sont fixées quatre baleines rabattables sur le corps dudit tube et pouvant s'en écarter en formant un parapluie, une extrémité du manchon en fil renfermant le tendon de banque à accrocher étant destiné à être passée dans ce tube et à en ressortir par un orifice ménagé au sommet de la tête ogivale, un noeud ou un système de sertissage empêchant le manchon et le tendon de faire le chemin inverse.

Conformément à l'invention, le corps du tube à tête ogivale comporte à sa partie haute, sous ladite tête, quatre ergots élastiques destinés à coopérer avec les baleines en s'accrochant dans la paroi osseuse de la perforation.

La mise en place dans l'os de l'ensemble constitué par le tube à tête ogivale et le tendon de banque gainé du manchon en fil est réalisée grâce à un tube intracteur comportant à sa partie haute quatre orifices pour le passage des ergots du tube à tête ogivale, après que ledit tube à tête ogivale et le tendon gainé du manchon aient été introduits dans ledit tube intracteur.

Les orifices ménagés dans le tube intracteur permettent d'éviter que les ergots soient écrasés contre la paroi interne du tube intracteur et qu'ils perdent leur élasticité, nécessaire pour leur accroche dans la perforation osseuse.

Ainsi, après que le tendon de banque ait été glissé dans le manchon en fil, l'ensemble est introduit dans le tube intracteur, une extrémité du manchon ressortant du côté de la partie haute du tube intracteur où sont situés les orifices pour le passage des ergots du tube à tête ogivale.

On passe ensuite cette extrémité du manchon dans un petit tube passe-fil permettant de la faire passer à travers l'orifice ménagé à l'extrémité du tube à tête ogivale. Le tube passe-fil est ensuite enlevé et l'extrémité du manchon dépassant du sommet de la tête ogivale nouée, de manière à empêcher son retrait.

En tirant légèrement sur la même extrémité du manchon on permet à une extrémité du tendon de banque de s'insérer dans le tube à tête ogivale, lequel s'insère dans le tube intracteur jusqu'à ce que les ergots viennent s'enclencher par élasticité dans les orifices dudit tube intracteur. Il ne reste plus au chirurgien qu'à glisser cet ensemble dans la perforation réalisée dans l'os, de telle sorte que la tête ogivale ressorte de l'autre côté de l'os dans une zone sous-tissulaire située sous la peau. Par traction sur le manchon, les baleines de la tête ogivale s'écartent grâce au noeud ou au système de sertissage qui appuie sur leur attache, et viennent en appui sur la zone osseuse périphérique de la perforation. Le tube intracteur est ensuite retiré.

La repousse de l'os permettra de coloniser l'ensemble inséré dans la perforation, ce qui assurera le maintien du tendon de banque lorsque le manchon en fil se sera résorbé.

Ce dispositif trouve également des applications dans les domaines de l'urologie, de la gynécologie et de la chirurgie digestive, permettant de remédier par exemple aux prolapsus, en utilisant notamment des tendons de banque renforcés de manchons selon l'invention, pour mieux suspendre les organes.

Les avantages et les caractéristiques de la présente invention ressortiront plus clairement de la description qui suit et qui se rapporte au dessin annexe, lequel en représente un mode de réalisation non limitatif.

Dans le dessin annexé :
- la figure 1 représente une vue en perspective d'un manchon de renforcement en fil résorbable selon l'invention.
- la figure 2a représente une vue en perspective partielle du même manchon enfilé sur un applicateur.
- la figure 2b représente une vue en perspective partielle du même manchon et du même applicateur dans lequel est introduit un transplant ou un tendon de banque.
- la figure 3 représente une vue en perspective partielle d'une manière de solidariser le transplant ou tendon de banque et le manchon à un point d'insertion ligamentaire.
- la figure 4 représente une vue en perspective du dispositif d'accrochage et d'intraction.
- la figure 5 représente une vue de dessus du tube à tête ogivale.
- la figure 6 représente une vue schématique en coupe d'un tube à tête ogivale disposé dans un os.

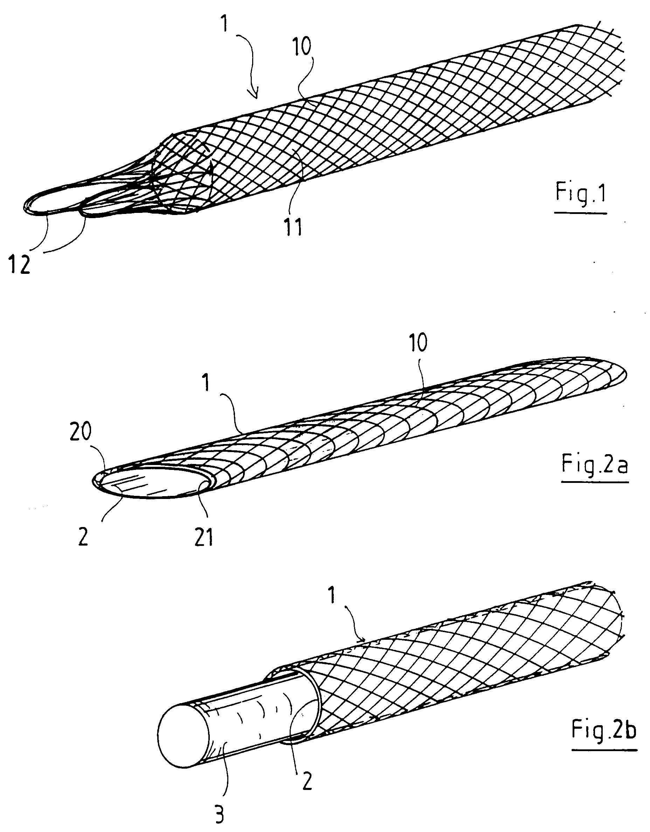

Si on se réfère à la figure 1 on peut voir que le dispositif de renforcement ligamentaire selon l'invention est constitué d'un manchon 1 tricoté au moyen d'un fil 10 en matériau résorbable. La maille 11, qui est de type connu, permet une extension dans le sens diamétral et une faible extension dans le sens longitudinal, quand le diamètre est fixé.

A l'une des extrémités du manchon 1, lui sont solidarisées deux boucles 12, constituées soit par le prolongement du fil 10 utilisé pour la confection dudit manchon 1, soit de fil, également résorbable, fixé solidement audit manchon 1.

Si on se réfère maintenant à la figure 2a on peut voir que le manchon 1 est conditionné, enfilé autour d'un applicateur 2 constitué d'un tube relativement souple susceptible d'être aplati selon deux génératrices diamétralement opposées, et créant deux plis 20 et 21.

Si on se réfère à la figure 2b on peut voir qu'après avoir exercé une pression sur l'applicateur 2, au niveau des plis 20 et 21, ledit applicateur 2 et le manchon 1 reprennent leur forme initiale cylindrique, et que l'on peut introduire à l'intérieur dudit applicateur 2 le transplant ou tendon de banque 3, de manière qu'après enlèvement dudit applicateur 2, le transplant ou tendon de banque 3 se trouve à l'intérieur du manchon 1, une traction sur les deux extrémités de celui-ci permettant d'en adapter le diamètre à celui du transplant ou tendon de banque 3.

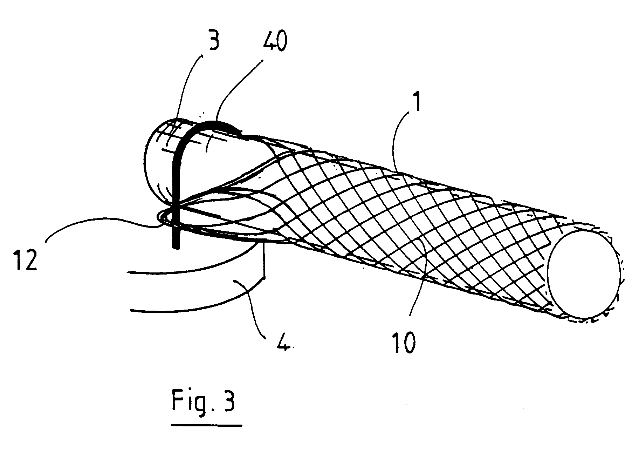

Si on se réfère maintenant à la figure 3 on peut voir que le transplant ou tendon de banque 3 gainé du manchon 1 est fixé sur l'os 4 de l'articulation au moyen d'une agrafe 40 emprisonnant d'une part le transplant ou le tendon de banque 3 et d'autre part les boucles 12 du manchon 1.

Il est également possible de réaliser cette solidarisation au moyen de plusieurs agrafes, l'une fixant le transplant ou tendon de banque 3 et deux autres fixant chacune une des boucles 12 du manchon de chaque côté dudit transplant ou tendon de banque 3.

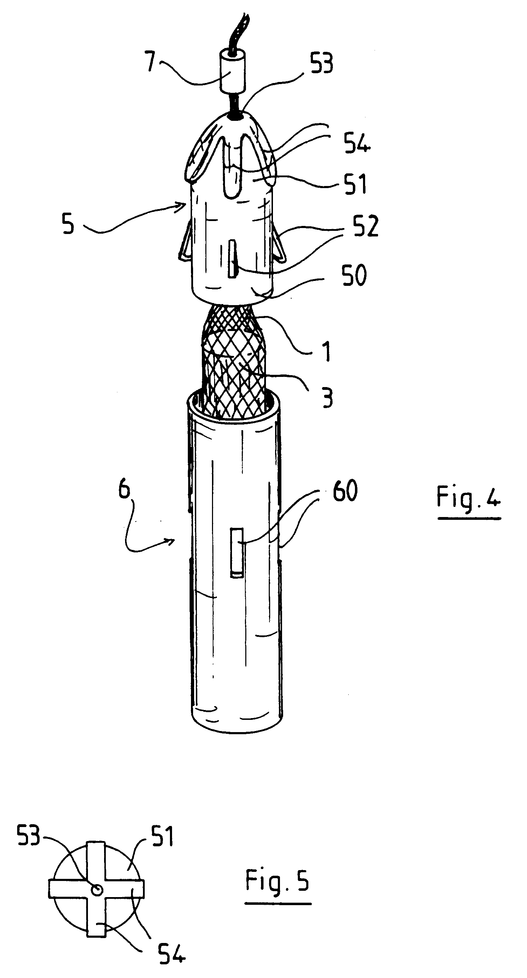

Si l'on se réfère à la figure 4 on peut voir un tendon de banque 3 glissé dans un manchon 1 en fil, cet ensemble ayant été inséré dans le tube intracteur 6.

L'extrémité du manchon 1 a été passée dans un tube passe-fil 7 avant d'être introduite dans le tube 5 à tête ogivale 51, de manière à pouvoir être passée à travers l'orifice 53 de la tête ogivale 51. Par la suite, le tube passe-fil 7 sera enlevé et l'extrémité du manchon 1 sera nouée afin qu'il ne puisse plus passer en sens inverse par l'orifice 53.

D'autre part, la tête ogivale 51 comporte des baleines 54 fixées par une de leurs extrémités autour de l'orifice 53 et possédant une certaine élasticité. Le corps 50 du tube 5 comporte, en partie haute et sous la tête ogivale 51, quatre ergots 52 rétractables placés dans l'alignement des baleines 54.

Le tube 5 à tête ogivale 51 est ensuite engagé dans le tube intracteur 6 jusqu'à ce que les quatre ergots 52 s'engagent par élasticité dans les orifices 60 ménagés dans le tube intracteur 6, les baleines 54 étant maintenues repliées par ce dernier.

La figure 5 représente, vue de dessus, la tête ogivale 51 avec le trou 53 et les quatre baleines 54 positionnées en croix et attachées à leur base autour dudit trou.

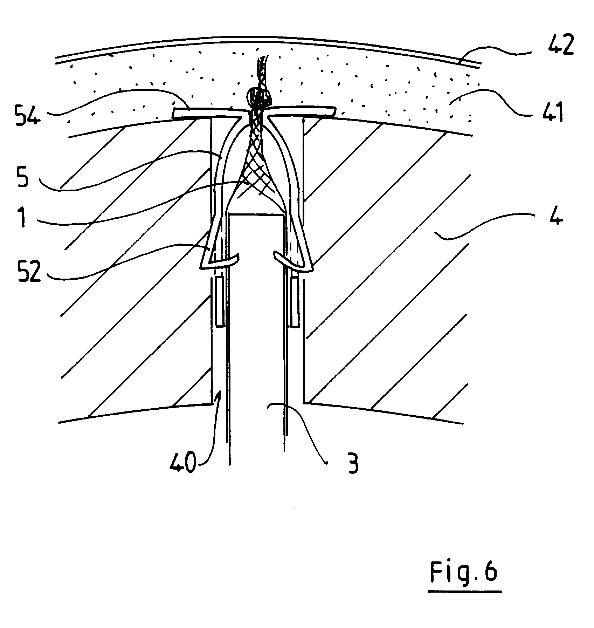

Si l'on se reporte à la figure 6 on peut voir un tube 5 inséré dans une perforation 40 d'un os du genou lors d'une intervention sur le tendon croisé du genou.

On peut remarquer que la perforation 40 n'a pas été faite en partant de la peau 42 située juste au-dessus de l'os 4, mais qu'elle a été pratiquée à partir d'un autre point.

L'avantage d'un tel procédé est que l'opération est réalisée en ne faisant qu'une seule incision au lieu des deux ou trois nécessitées par les procédés actuels.

La mise en place du tube 5 à tête ogivale 51 et du tendon de banque 3 gainé du manchon 1 est réalisée grâce au tube intracteur 6 qu'on insère dans la perforation 40 pratiqué dans l'os 4.

Après que le tube intracteur 6 ait été inséré dans l'orifice 40, il suffit de le retirer pour que, du fait de leur élasticité, les baleines 54 et les ergots 52 ainsi libérés s'écartent pour prendre appui sur la partie osseuse, renforçant ainsi la fixation du tendon 3.

En opérant une traction sur le manchon 3, les ergots 52 s'accrochent à la paroi osseuse de la perforation 40 et coopèrent ainsi avec les baleines 54, leur extrémité libre dans le tube servant en outre à l'ancrage du tendon, réduisant ainsi l'effort exercé sur le noeud au sertissage de l'extrémité 1 du manchon 3.

Le tube 5 à tête ogivale peut être réalisé en métal ou en polyéthylène.

D'autre part, un bloc osseux peut être laissé à l'extrémité du tendon de banque ou du transplant pour faciliter la prolifération osseuse et l'ancrage dudit tendon ou transplant.

## Revendications

1. Dispositif de renforcement, au cours d'une plastie ligamentaire, d'un transplant constitué d'un manchon (1) tricoté dans un fil (10) en matériau résorbable dont la maille (11) permet une extension dudit manchon (1) essentiellement dans le sens diamétral tout en autorisant les échanges biologiques entre le transplant (3) et le milieu environnant, caractérisé en ce que le manchon (1) est conditionné sur un applicateur (2) constitué d'un tube de matière souple susceptible d'être aplati selon deux génératrices diamétralement opposées (20, 21).

2. Dispositif selon la revendication 1, caractérisé en ce que le manchon (1) est associé à un tube (5) à tête ogivale (51) adapté à être glissé dans un tube intracteur (6) dans lequel le transplant (3) gainé du manchon (1) a été préalablement placé, l'extrémité du manchon (1) étant passée, au moyen d'un tube passe-fil (7), à travers l'orifice (53) de la tête ogivale (51), laquelle comporte des baleines (54) présentant une certaine élasticité, fixées par une de leurs extrémités autour dudit orifice (53), rabattables sur le corps dudit tube (5) sur lequel elles restent appliquées au moyen du tube intracteur (6) pendant la mise en place dans l'os du tube (5) à tête ogivale (51), le retrait du tube intracteur (6) permettant ensuite aux baleines (54) de s'écarter et de prendre appui sur la zone périphérique de la perforation osseuse (40), tandis que le tube passe-fil (7) est enlevé et que l'extrémité du manchon (1) dépassant l'orifice (53) de la tête ogivale (51) est nouée.

3. Dispositif selon la revendication 2 caractérisé en ce que le corps du tube (5) comporte en partie haute quatre ergots élastiques (52) susceptibles de s'insérer dans quatre orifices (60) ménagés dans le tube intracteur (6) et destinés à coopérer avec les baleines (54) en s'accrochant à la paroi osseuse de la perforation (40) réalisée dans l'os.

## Claims

1. Device for reinforcing during ligamentary plastic surgery, a transplant consisting of a sleeve (1) which is knitted from a thread (10) made of reabsorbable material, the mesh (11) of which permits extension of the said sleeve (1) substantially in the diametral direction, at the same time allowing biological exchanges between the transplant (3) and the surrounding environment, characterised in that the sleeve (1) is packed on an applicator (2) which consists of a tube of flexible material which can be flattened according to two diametrically opposed generatrices (20, 21).

2. Device according to Claim 1, characterised in that the sleeve (1) is associated with a tube (5) which has an ogival head (51) which can be slid in an interaction tube (6) in which the sheathed transplant (3) of the sleeve (1) has previously been placed, the end of the sleeve (1) being passed by means of a thread-guide tube (7) through the aperture (53) in the ogival head (51) which includes small rods (54) which have specific resilience, are attached by one of their ends around the said aperture (53), and can be folded onto the body of the said tube (5), to which they remain attached by means of the interaction tube (6) whilst the ogival head (51) tube (5) is being inserted in the bone, withdrawal of the interaction tube (6) then allowing the small rods (54) to move apart and to be supported on the peripheral area of the perforation of the bone (40), whereas the thread-guide tube (7) is removed and the end of the sleeve (1) which extends beyond the aperture (53) of the ogival head (51) is knotted.

3. Device according to Claim 2, characterised in that the body of the tube (5) contains on its upper part four resilient lugs (52) which can be inserted in four apertures (60) provided in the interaction tube (6) and which can cooperate with the small rods (54) by adhering to the osseous wall of the perforation (40) provided in the bone.

## Patentansprüche

1. Vorrichtung zum Verstärken eines Transplantates im Verlaufe einer Wiederherstellungschirurgie, umfassend eine Manschette (1), die aus einem Faden (10), der aus resorbierbarem Material gestrickt ist und dessen Masche (11) eine Ausdehnung der Manschette (1) im wesentlichen in diametraler Richtung erlaubt und gleichzeitig einen biologischen Austausch zwischen dem Transplantat (3) und der Umgebung zuläßt, dadurch gekennzeichnet, daß die Manschette (1) auf einen Applikator (2) aufgebracht ist, der aus einer Hülse aus weichem Material besteht, die entlang zweier einander gegenüberliegender Mantellinie (20, 21) flachgelegt werden kann.

2. Vorrichtung nach Anspruch 1, dadurch gekennzeichnet, daß die Manschette einer Hülse (5) mit einem spitzbogenförmigen Kopf (51) zugeordnet ist, der derart gestaltet ist, daß er in eine Zwischenhülse (6) hineingleiten kann, in welcher das Transplantat (3), das von der Manschette (6) überzogen ist, zuvor eingesetzt wurde, daß das Ende der Manschette (1) mittels einer Fadeneinführungstülle (7) durch die Öffnung (53) des spitzbogenförmigen Kopfes (51) hindurchgeführt ist, welcher Rippen (54) aufweist, die eine gewisse Elastizität haben und die mit einem ihrer Enden um die Öffnung (53) herum fixiert und an die Hülse (5) heranklappbar sind, auf welcher sie mittels der Zwischenhülse (6) während des Einsetzens der Hülse (5) mit spitzbogenförmigem Kopf (51) in den Knochen verbleiben, und daß das Herausziehen der Zwischenhülse (6) sodann ein Aufspreizen der Rippen (54) und ein Anlegen an die Umfangszone der Knochenbohrung (40) erlaubt, während die Fadeneinführungstülle (7) abgehoben und das die Öffnung (53) des spitzbogenförmigen Kopfes (51) durchlaufende Ende der Manschette (1) verknotet wird.

3. Vorrichtung nach Anspruch 2, dadurch gekennzeichnet, daß der Körper der Hülse (5) in seinem oberen Teil vier elastische Nocken (52) aufweist, die in vier Öffnungen (60) in der Zwischenhülse (6) eingreifen können, und die dazu dienen, mit den Rippen (54) zusammenzuarbeiten, indem sie sich in die Wandung der in den Knochen eingebrachten Bohrung (40) einhaken.
